(19) Europäisches Patentamt
European Patent Office
Office européen des brevets

(11) Veröffentlichungsnummer: **0 069 954**
**B1**

(12) # EUROPÄISCHE PATENTSCHRIFT

(45) Veröffentlichungstag der Patentschrift: **05.12.84**

(51) Int. Cl.³: **C 07 C 69/743,** C 07 C 67/03

(21) Anmeldenummer: **82105966.4**

(22) Anmeldetag: **05.07.82**

(54) **Verfahren zur Herstellung von Permethrinsäurementhylester.**

(30) Priorität: **14.07.81 DE 3127752**

(43) Veröffentlichungstag der Anmeldung: **19.01.83 Patentblatt 83/3**

(45) Bekanntmachung des Hinweises auf die Patenterteilung: **05.12.84 Patentblatt 84/49**

(84) Benannte Vertragsstaaten: **BE CH DE FR GB IT LI NL**

(56) Entgegenhaltungen:
**EP - A - 0 022 972**
**DE - A - 2 716 771**
**DE - A - 2 820 521**
**DE - A - 2 843 073**

(73) Patentinhaber: **BAYER AG, Konzernverwaltung RP Patentabteilung, D-5090 Leverkusen 1 Bayerwerk (DE)**

(72) Erfinder: **Sehnem, Hans Peter, Dipl.-Ing., Nüller Strasse 90, D-5600 Wuppertal 1 (DE)**

EP 0 069 954 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Permethrinsäurementhylester.

Es ist bereits bekannt, dass man Permethrinsäurementhylester erhält, indem man Permethrinsäureethylester mit Menthol erhitzt. Die dabei erhältlichen Ausbeuten sind jedoch nicht zufriedenstellend (DE-OS Nr. 2928406). Dies, obwohl mit Titantetraethylat als Umesterungskatalysator gearbeitet wird. Titantetraalkohole gelten für derartige Umesterung als besonders geeignet (DE-OS Nr. 2812365).

Umesterungsreaktionen von Permethrinsäuremethylester mit Natriummethylat als Umesterungkatalysator sind bereits bekannt. Es muss dabei jedoch in Lösungsmittel gearbeitet werden, um das entstehende Methanol abdestillieren zu können. Die Ausbeuten und Reinheiten bei der Umesterung sind nicht befriedigend (DE-OS Nr. 2716771).

Es wurde ein Verfahren zur Herstellung von Permethrinsäurementhylester der Formel I gefunden,

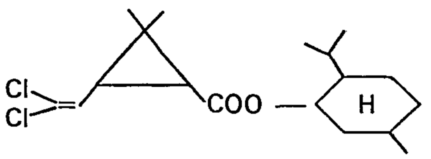 (I)

indem man Permethrinsäuremethyl- oder -ethylester mit Menthol umsetzt, das dadurch gekennzeichnet ist, dass man in Gegenwart von 1 bis 30 Mol-% Alkalialkoholat, gegebenenfalls in Form seiner alkoholischen Lösungen, bezogen auf den eingesetzten Permethrinsäuremethyl- oder -ethylester zwischen 50 und 180°C arbeitet.

Formel I umfasst dabei sämtliche Enantiomeren.

Es war überraschend, dass die Umesterung in Gegenwart von Alkalialkoholaten ohne Bildung der Chloracetylenverbindung der Formel

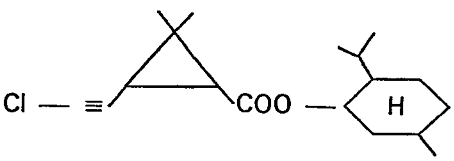

abläuft.

Es war ausserdem überraschend, dass es mit Hilfe von Alkalialkoholat möglich ist, die Ausbeute der Umesterung im Vergleich zu den als besonders gut wirksam geltenden Umesterungskatalysatoren Titantetraalkoholat beträchtlich zu steigern. Die Ausbeutesteigerung ist auch im Hinblick darauf überraschend, dass ohne die Verwendung von Lösungsmitteln gearbeitet wird, die dem entstehenden Alkohol azeotrop aus dem Reaktionsgemisch schleppen.

Die erfindungsgemässe Reaktion wird bei Temperaturen zwischen 50 und 180, bevorzugt zwischen 80 und 140°C, durchgeführt.

Bevorzugt werden als Alkalialkoholate Natriummethylat und Natriumethylat eingesetzt. Die Alkalialkoholate werden in einer Menge von 1 bis 30, bevorzugt 2 bis 20 Mol-%, bezogen auf die eingesetzte Permethrinsäureestermenge, eingesetzt. Die Alkoholate können als solche oder in Form ihrer alkoholischen Lösungen eingesetzt werden. Bevorzugt sind die alkoholischen Lösungen der Alkalialkoholate.

Pro Mol Permethrinsäureester werden 1 bis 5, bevorzugt 1,1 bis 2,5 mol Menthol eingesetzt.

Menthol wird sowohl in Form des d- als auch des l-Enantiomeren eingesetzt.

Das Verfahren kann dabei kontinuierlich oder diskontinuierlich durchgeführt werden.

Das Verfahren wird ohne die Verwendung besonderer Lösungsmittel durchgeführt. Diese Arbeitsweise hat den Vorteil, dass bei der späteren Trennung der Permethrinsäurementhylester in ihre optisch aktiven Isomeren diese Lösungsmittel nicht entfernt werden müssen.

Die nach dem Verfahren erhältlichen Menthylester dienen dazu, die optischen Isomeren der Permethrinsäure zu trennen. Dazu wird das anfallende d-Menthylestergemisch mit einem geeigneten Lösungsmittel versetzt und der (+)-Transpermethrinsäure-d-menthylester abgesaugt.

Geeignete Lösungsmittel sind Kohlenwasserstoffe wie Ligroin, Petroether, Hexan, Heptan, Pentan, Cyclohexan, sowie Ketone wie Aceton, Methylethyketon, Methylisobutylketon; Ester wie Aminensäuremethyl oder -ethylester, Essigsäuremethyl oder -ethylester, Propionsäuremethyl oder -ethylester; sowie Alkohole wie Methanol, Ethanol, Isopropanol, n-Propanol, Butanol; besonders bevorzugt wird Ligroin, Methanol, Ethanol.

Eine andere Möglichkeit, das anfallende Menthylestergemisch in seine optisch aktiven Isomeren zu trennen, besteht darin, dass man ohne Lösungsmittel arbeitet. Dazu wird das in Form eines Öls anfallende Estergemisch auf 25 bis 50°C, bevorzugt Raumtemperatur, abgekühlt und das dabei auskristallisierende, schwerer lösliche (+)-Transisomere abgesaugt.

*Beispiel 1*

In einem Rührkolben werden 1 mol (223 g) (±)-Transpermethrinsäuremethylester, 195 g d-Menthol (1,25 mol) und 7,3 g 11%ige Natriummethylatlösung (0,035 mol) im Vakuum von ca. 50 mbar auf 110 bis 120°C erhitzt.

Nach 1stündiger Nachrührzeit bei 110 bis 120°C wird 1 h bei ca. 5 mbar und 110 bis 120°C weitergerührt, wobei überschüssiges Menthol abdestilliert. Es verbleiben 351 g einer hellgelben Schmelze, die beim Abkühlen erstarrt, mit einer Zusammensetzung von 95,2% (±)-Transpermethrinsäure-d-menthylester, 1,8% d-Menthol, 1,9% (±)-Transpermethrinsäuremethylester und <0,2% 2,2-Dimethyl-3-(2-chlorethinyl)cyclopropancarbonsäure-d-menthylester.

Bei Behandlung der Schmelze mit einem geeigneten Lösungsmittel können die beiden Enantiomeren voneinander getrennt werden.

*Beispiel 2*

Über einen Dünnschichtverdampfer (Länge des Verdampferteils ca. 200 mm; ⌀ ca. 30 mm) gibt man im Verlaufe 1 h unter Vakuum von ca. 5 mbar

und einer Heizmanteltemperatur von 110°C eine auf ca. 50°C erwärmte Mischung aus 223 g (±)-Transpermethrinsäuremethylester (1 mol), 195 g d-Menthol (1,25 mol) und 7,3 g 11%ige Natriummethylatlösung (0,035 mol). Als Sumpf erhält man 345,2 g einer gelblichen bis beigen Schmelze mit einer Zusammensetzung von 96,3% (±)-Transpermethrinsäure-d-menthylester, 0,9% d-Menthol, 1,3% (±)-Transpermethrinsäuremethylester und <0,2% 2,2-Dimethyl-3-(2-chlorethinyl)cyclopropancarbonsäure-d-menthylester.

*Beispiel 3*

In einem Rührkolben werden 1 mol (223 g) (±)-Transpermethrinsäuremethylester, 195 g d-Menthol (1,25 mol) und 7,3 g 11%ige Natriummethylatlösung (0,035 mol) im Vakuum von ca. 50 mbar auf 110 bis 120°C erhitzt.

Nach 1stündiger Nachrührzeit bei 110 bis 120°C wird 1 h bei ca. 5 mbar und 110 bis 120°C weitergerührt, wobei überschüssiges Menthol abdestilliert. Nach Zugabe von 500 ml Methanol wird der Kolbeninhalt auf 15°C abgekühlt, das ausgefallene Kristallisat abgesaugt, zweimal mit jeweils 125 ml Methanol gewaschen und anschliessend im Vakuum getrocknet.

Man erhält 143,9 g (+)-Transpermethrinsäure-d-menthylester mit einem Gehalt von 99%.

*Beispiel 4*

In einem Rührkolben werden 223 g (1 mol) (±)-Transpermethrinsäuremethylester, 312 g d-Menthol (2 mol) vorgelegt und auf 130 bis 140°C erhitzt. Bei 130 bis 140°C werden im Vakuum von 300 mbar 5 g 11%ige Natriummethylatlösung zugetropft und 30 min 130 bis 140°C und ca. 300 mbar nachgerührt, wobei Methanol abdestilliert. Nach Abkühlung auf ca. 60 bis 65°C werden 500 ml Methanol zugegeben und das Reaktionsgemisch auf 5°C abgekühlt, wobei der (±)-Transpermethrinsäure-d-menthylester auskristallisiert.

Das Kristallisat wird abgesaugt, zweimal mit jeweils 100 ml Methanol gewaschen und anschliessend im Vakuum getrocknet. Man erhält 132,7 g (±)-Transpermethrinsäure-d-menthylester mit einem Gehalt von 99,3%.

**Patentanspruch**

Verfahren zur Herstellung von Permethrinsäurementhylester der Formel

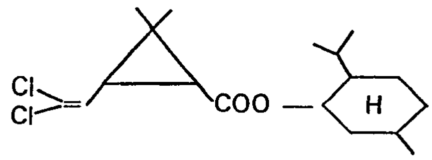

indem man Permethrinsäuremethyl- oder -ethylester mit Menthol umsetzt, dadurch gekennzeichnet, das man in Gegenwart von 1 bis 30 Mol-% Alkalialkoholat, gegebenenfalls in Form seiner alkoholischen Lösungen, bezogen auf den eingesetzten Permethrinsäuremethyl- oder -ethylester zwischen 50 und 180°C arbeitet.

**Claim**

Process for the preparation of permethric acid menthyl ester of the formula

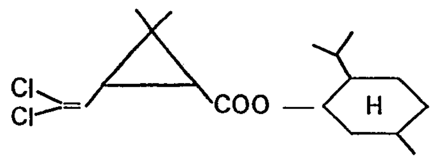

by reacting permethric acid methyl or ethyl ester with menthol, characterised in that the reaction is carried out in the presence of 1 to 30 mol-% of an alkali metal alcoholate, if appropriate in the form of its alcoholic solutions, based, on the permethric acid methyl or ethyl ester used, at between 50 and 180°C.

**Revendication**

Procédé de production de l'ester de menthyle de l'acide perméthrique de formule

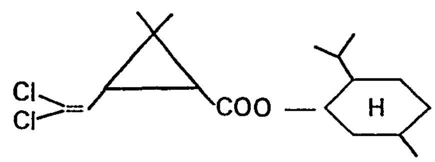

par réaction de l'ester de méthyle ou d'éthyle de l'acide perméthrique avec le menthol, caractérisé en ce qu'on opère entre 50 et 180°C en présence de 1 à 30 mol-% d'un alcoolate alcalin, éventuellement sous la forme de ses solutions alcooliques, par rapport à l'ester méthylique ou éthylique d'acide perméthrique utilisé.